# EUROPEAN PATENT APPLICATION

(11) **EP 4 079 763 A1**
(43) Date of publication of application: **26.10.2022**
(21) Application number: 20901873.8
(22) Date of filing: 18.12.2020
(51) Int. Cl.: C07K 16/28, C12N 15/13, A61K 39/395, A61P 35/00

(54) **ANTI-HUMAN PROGRAMMED DEATH -1 (PD-1) MONOCLONAL ANTIBODY**

(30) Priority: 20.12.2019 CN 201911342193
(71) Applicant: Guangdong Feipeng Pharmaceutical Co., Ltd, Dongguan, Guangdong 523808 (CN)
(72) Inventor: LU, Lisheng, Hong Kong Cooperation Zone Shenzhen, Guangdong 518052 (CN); HUO, Yongting, Hong Kong Cooperation Zone Shenzhen, Guangdong 518052 (CN); FU, Jun, Hong Kong Cooperation Zone Shenzhen, Guangdong 518052 (CN); LI, Fan, Hong Kong Cooperation Zone Shenzhen, Guangdong 518052 (CN); GONG, Chunxi, Hong Kong Cooperation Zone Shenzhen, Guangdong 518052 (CN); PAN, Zhifu, Hong Kong Cooperation Zone Shenzhen, Guangdong 518052 (CN)
(74) Representative: Biggi, Cristina
(86) International application number: PCT/CN2020/137548
(87) International publication number: WO 2021/121373

(57) **Abstract**

Provided are a human PD-1 antibody, an antigen-binding fragment thereof and a medical use thereof. A chimeric antibody containing a complementarity determining region (CDR) of the antibody, a pharmaceutical composition containing the human PD-1 antibody and the antigen-binding fragment thereof, and a use of the antibody in preparation of a drug for treating a disease or a disorder are further disclosed.

## Description

### Technical Field

The present application relates to the field of immunology, in particular to a mouse anti-human programmed death-1 (PD1) monoclonal antibody and an application thereof.

### Background

PD1 is a member of a CD28 family, and is expressed on activated B cells, T cells and myeloid cells. Human PD1 is encoded by a gene Pdcd1, and located at 2q37.3, with a full length of 9.6 kb, consisting of 5 exons and 4 introns, and its upstream contains a 663 bp promoter. PD1 is a 55 KDa type-I transmembrane protein, and the molecular structure is formed by an extracellular region, a transmembrane region and an intracellular region. The extracellular region contains an immunoglobulin variable region IgV domain, and the intracellular region contains an immunoreceptor tyrosine inhibitory motif (ITIM) and an immunoreceptor tyrosine switching module (ITSM). An amino acid sequence of the PD-1 extracellular region has 24% of the homology with a cytotoxic T lymphocyte-associated antigen-4 (CTLA-4), and 28% of the homology with CD28. After T cells are activated, PD-1 assembles a tyrosine phospholipase SHP2 mainly through ITIM, leading to dephosphorylation of a downstream effector molecule.

PD-1 has two ligands: PD-L1 and PD-L2. Both PD-L1 and PD-L2 are B7 homologues. A PDL gene is located at a 9P24.2 site of a human chromosome, and is 42 kb in size. Both molecular structures contain an immunoglobulin-like variable region domain, a constant region-like domain, a transmembrane region and a short cytoplasmic tail.

After PD-1 binds to PD-L1 and PD-L2, the activation of T cells may be down-regulated. PD-L1 is expressed on the surface of a variety of tumor cells, and these tumor cells include: a lung cancer, a gastric cancer, a liver cancer, an esophageal cancer, a kidney cancer, an ovarian cancer, a cervical cancer, a breast cancer, a skin cancer, a colon cancer, a bladder cancer, a glial cancer, a head and neck cancer, and an oral squamous cell cancer. Moreover, a large number of CD8+T cells expressing PD-L1 are found around these cancers. Clinical result statistics show that a high expression level of PD-L1 on the tumor cells is related to poor prognosis of a cancer patient.

### Summary

A technical problem to be solved by the present invention is to overcome defects and deficiencies that an existing antibody has the low affinity and specificity for binding to PD-1 and may not effectively block the binding of PD-1 to PD-L1 and PD-L2 and provide a new antibody that may efficiently bind to PD-1 and may block the binding of PD-1 to PD-L1 and PD-L2. A mouse monoclonal antibody may better promote T cells to secrete cytokines IL2 and IFNγ in staphylococcal enterotoxin B (SEB) superantigen stimulation of a peripheral blood mononuclear cell (PBMC) and a mixed lymphocyte reaction (MLR).

The present invention provides an anti-PD1 monoclonal antibody. Specifically, the present invention provides the following aspects:
1. An anti-PD1 antibody or an antigen-binding fragment thereof, comprising a heavy chain CDR1-3 and a light chain CDR1-3 selected from a group consisting of the following items:
   (1) HCDR1: represented by a sequence of SEQ ID NO: 8; HCDR2: represented by a sequence of SEQ ID NO: 9; HCDR3: represented by a sequence of SEQ ID NO: 10;
      LCDR1: represented by a sequence of SEQ ID NO: 20; LCDR2: represented by a sequence of SEQ ID NO: 21; LCDR3: represented by a sequence of SEQ ID NO: 22;
   (2) HCDR1: represented by a sequence of SEQ ID NO: 12; HCDR2: represented by a sequence of SEQ ID NO: 13; HCDR3: represented by a sequence of SEQ ID NO: 14;
      LCDR1: represented by a sequence of SEQ ID NO: 24; LCDR2: represented by a sequence of SEQ ID NO: 25; LCDR3: represented by a sequence of SEQ ID NO: 26; and
   (3) HCDR1: represented by a sequence of SEQ ID NO: 16; HCDR2: represented by a sequence of SEQ ID NO: 17; HCDR3: represented by a sequence of SEQ ID NO: 18;
      LCDR1: represented by a sequence of SEQ ID NO:28; LCDR2: represented by a sequence of SEQ ID NO:29; LCDR3: represented by a sequence of SEQ ID NO:30.
2. The antibody or antigen-binding fragment thereof of the above 1, herein the antibody includes:
   (1) a heavy chain variable region, herein it includes or consists of the following sequences:
      an amino acid sequence shown in SEQ ID NO: 7, or
      a sequence having at least 60%, 70%, 80%, and 85%, preferably at least 86%, 87%, 88%, 89%, 90%, 91%, 92%, 93%, 94%, 95%, 96%, 97%, 98% or 99% of the sequence identity with the sequence shown in SEQ ID NO: 7, and
      a light chain variable region, herein it includes or consists of the following sequences:
      an amino acid sequence shown in SEQ ID NO: 19, or
      a sequence having at least 60%, 70%, 80%, and 85%, preferably at least 86%, 87%, 88%, 89%, 90%, 91%, 92%, 93%, 94%, 95%, 96%, 97%, 98% or 99% of the sequence identity with the sequence shown in SEQ ID NO: 19;
   (2) a heavy chain variable region, herein it includes or consists of the following sequences:
      an amino acid sequence shown in SEQ ID NO: 11, or
      a sequence having at least 60%, 70%, 80%, and 85%, preferably at least 86%, 87%, 88%, 89%, 90%, 91%, 92%, 93%, 94%, 95%, 96%, 97%, 98% or 99% of the sequence identity with the sequence shown in SEQ ID NO: 11, and
      a light chain variable region, herein it includes or consists of the following sequences:
         an amino acid sequence shown in SEQ ID NO: 23, or
         a sequence having at least 60%, 70%, 80%, and 85%, preferably at least 86%, 87%, 88%, 89%, 90%, 91%, 92%, 93%, 94%, 95%, 96%, 97%, 98% or 99% of the sequence identity with the sequence shown in SEQ ID NO: 23; and
   (3) a heavy chain variable region, herein it includes or consists of the following sequences:
      an amino acid sequence shown in SEQ ID NO: 15, or
      a sequence having at least 60%, 70%, 80%, and 85%, preferably at least 86%, 87%, 88%, 89%, 90%, 91%, 92%, 93%, 94%, 95%, 96%, 97%, 98% or 99% of the sequence identity with the sequence shown in SEQ ID NO: 15, and
      a light chain variable region, herein it includes or consists of the following sequences:
         an amino acid sequence shown in SEQ ID NO: 27, or
         a sequence having at least 60%, 70%, 80%, and 85%, preferably at least 86%, 87%, 88%, 89%, 90%, 91%, 92%, 93%, 94%, 95%, 96%, 97%, 98% or 99% of the sequence identity with the sequence shown in SEQ ID NO: 27.
3. The antibody or antigen-binding fragment thereof of any one of the above 1-2, herein the antibody further includes a heavy chain constant region and a light chain constant region, preferably derived from human IgG or IgM, more preferably IgG4.
4. The antibody or antigen-binding fragment thereof of any one of the above 1-2, herein the antigen-binding fragment is selected from Fab, scFv, Fab', dAb, F(ab')₂, Fv or Fab/c.
5. The antibody or antigen-binding fragment thereof of any one of the above 1-2, herein the antibody is a chimeric antibody or a multispecific antibody (for example, a bispecific antibody).
6. A polynucleotide encoding the antibody or antigen-binding fragment thereof of any one of the above 1-5.
7. A pharmaceutical composition, herein it contains the antibody or antigen-binding fragment thereof of any one of the above 1-5; and optionally, it further includes a pharmaceutically acceptable carrier and/or excipient.
8. The PD-1 antibody or antigen-binding fragment thereof of any one of the above 1-5, and a use of the pharmaceutical composition of the above 7 in preparation of a drug for treating a PD-1-mediated disease or disorder.
9. The antibody or antigen-binding fragment thereof of any one of the above 1-5, and a use of the pharmaceutical composition of the above 7 in prevention and/or treatment and/or adjuvant treatment and/or diagnosis treatment of a tumor disease or in preparation of a drug for the prevention and/or treatment and/or adjuvant treatment and/or diagnosis treatment of the tumor disease, preferably, the tumor is selected from a lung cancer, a gastric cancer, a liver cancer, an esophagus cancer, a kidney cancer, an ovarian cancer, a cervical cancer, a breast cancer, a skin cancer, a colon cancer, a bladder cancer, a glial cancer, a head and neck cancer, and an oral squamous cell cancer.
10. The pharmaceutical composition of the above 7 or the use of the above 9, herein the pharmaceutical composition or drug is in a form suitable for injection, preferably the form suitable for administration by subcutaneous injection, intradermal injection, intravenous injection, intramuscular injection or intralesional injection.

As used in the present invention, a term "light chain" includes a full-length light chain and a fragment thereof having a variable region sequence to confer binding specificity. The full-length light chain includes a variable region domain V_{L} and a constant region domain C_{L}. The variable region domain of the light chain is at an amino terminus of a polypeptide. The light chain includes a k chain and a λ chain.

As used in the present invention, a term "heavy chain" includes a full-length heavy chain and a fragment thereof having a variable region sequence to confer binding specificity. The full-length heavy chain includes a variable region domain V_{H} and three constant region domains C_{H1}, C_{H2} and C_{H3}. The V_{H} domain is at an amino terminus of a polypeptide, and the C_{H} domain is at a carboxy terminus, C_{H3} is closest to the carboxy terminus of the polypeptide. The heavy chain may be of any isotypes, including IgG (including IgGI, IgG2, IgG3, and IgG4 subtypes), IgA (including IgAI and IgA2 subtypes), IgM, and IgE.

As used in the present invention, a term "Fab fragment" consists of one light chain and C_{H1} and a variable region of one heavy chain. The heavy chain of a Fab molecule may not form a disulfide bond with another heavy chain molecule.

As used in the present invention, a term "Fc" region contains two heavy chain fragments including C_{H1} and C_{H2} domains of an antibody. The two heavy chain fragments are kept together by two or more disulfide bonds and by a hydrophobic interaction of a C_{H3} domain.

As used in the present invention, a term "Fab' fragment" contains a part of one light chain and one heavy chain (it contains a V_{H} domain and a C_{H1} domain and also a part of a region between the C_{H1} and C_{H2} domains), so that an interchain disulfide bond may be formed between two heavy chains of two Fab' fragments to form an F(ab')₂ molecule.

As used in the present invention, a term "F(ab')₂ fragment" contains two light chains and two heavy chains containing a part of a constant region between the C_{H1} and C_{H2} domains, so that an interchain disulfide bond is formed between the two heavy chains. The F(ab')₂ fragment thus consists of two Fab' fragments kept together by the disulfide bond between the two heavy chains.

As used in the present invention, a term "Fv region" includes a variable region from a heavy chain and a light chain, but lacks a constant region.

As used in the present invention, a term "Fd" fragment means an antibody fragment consisting of V_{H} and C_{H1} domains (Ward et al., Nature 341:544-546 (1989)).

As used in the present invention, a term "dAb" fragment (Ward et al., Nature 341:544-546 (1989)) consists of a V_{H} domain.

As used in the present invention, a term "Fab/c" fragment is an intermediate product of antibody cleavage formed by pepsin digestion of an immunoglobulin, it has the advantages of both Fab and Fc regions, namely it has the characteristics of strong diffusivity and slow metabolic clearance in vivo, and may maintain a high affinity (Liu Jianjun, "Journal of Cell and Molecular Immunology", 1989 (4): 29-29).

As used in the present invention, a term "single chain antibody" is an Fv molecule in which heavy and light chain variable regions are linked by a flexible linker to form a single polypeptide chain (it forms the antigen-binding region) (referring to, for example, Bird et al., Science. 242:423-426(1988) and Huston et al., Proc. Natl. Acad. Sci. USA. 90:5879-5883 (1988)).

As used in the present invention, a term "domain antibody" is an immunofunctional immunoglobulin fragment containing only a variable region of a heavy chain or a variable region of a light chain, including a multivalent domain antibody or a bivalent domain antibody. In some cases, two or more V_{H} regions are covalently linked by a peptide linker to generate the multivalent domain antibody (especially the bivalent domain antibody). Two V_{H} regions of the bivalent domain antibody may target the same or different antigens.

As used in the present invention, a term "multispecific antigen-binding protein" or "multispecific antibody" is an antigen-binding protein or antibody that targets more than one antigen or epitope.

As used in the present invention, a term "bispecific", "dual specific" or "bifunctional" antigen-binding protein or antibody is a hybrid antigen-binding protein or antibody that has two different antigen-binding sites respectively. The bispecific antibody is a multispecific antigen-binding protein or multispecific antibody, and may be generated by a variety of methods, including, but not limited to, fusion of hybridomas or linkage of Fab' fragments. Referring to, for example, Songsivilai and Lachmann, 1990, Clin. Exp. Immunol. 79:315-321; Kostelny et al., 1992, J. Immunol. 148:1547-1553. The two binding sites of the bispecific antigen-binding protein or antibody may bind two different epitopes, and the epitopes are present on the same or different protein targets.

As used in the present invention, a term "epitope" refers to a site on an antigen that is specifically bound by an immunoglobulin or antibody. The "epitope" is also known in the field as an "antigenic determinant". The epitope or antigenic determinant usually consists of a chemically active surface group of a molecule such as an amino acid or a carbohydrate or a sugar side chain and usually has the specific three-dimensional structural characteristics as well as the specific charge characteristics. For example, the epitope generally includes at least 3, 4, 5, 6, 7, 8, 9, 10, 11, 12, 13, 14 or 15 continuous or non-continuous amino acids in a unique spatial conformation, and it may be "linear" "or conformational". Referring to, for example, Epitope Mapping Protocols in Methods in Molecular Biology, Vol. 66, G.E. Morris, Ed. (1996). In a linear epitope, all points of interaction between a protein and an interacting molecule (for example, an antibody) exist linearly along a primary amino acid sequence of the protein. In a conformational epitope, points of interaction exist across protein amino acid residues that are separated from each other.

As used herein, terms "similarity" or "sequence similarity", and "identity" refer to relationships between sequences of two or more proteins or polypeptide molecules, for example, it is determined by aligning and comparing the sequences. "Percent identity" means a percentage of an identical residue between amino acids in molecules being compared and may be calculated on the basis of the size of the smallest molecule to be compared. In order to perform these calculations, a gap (if any) in the alignment must be solved by a specific mathematical model or a computer program (namely, an "algorithm"). While applied to the polypeptide, a term "substantially identity" refers to two peptide sequences that share at least 70%, 75% or 80% of the sequence identity, at least 90% or 95% of the sequence identity, and at least 97%, 98% or 99% of the sequence identity while optimally aligned, for example, with a program GAP or BESTFIT, by using a default gap weight provided by the program. In some cases, a difference of different residue sites is conservative amino acid substitution. The "conservative amino acid substitution" is such a substitution in which an amino acid residue is substituted with another amino acid residue having a side chain R group with similar chemical properties (for example, charge or aqueous). Generally, the conservative amino acid substitution may not substantially change the functional properties of the protein. In the case that two or more amino acid sequences differ from each other by the conservative substitution, the percent sequence identity may be up-regulated to correct for the conservative nature of the substitution. A method for making this adjustment is well-known to those skilled in the art. Referring to, for example, Pearson, Methods Mol. Biol. 243:307-31 (1994). Examples of an amino acid group having a side chain with the similar chemical properties include 1) aliphatic hydroxyl side chain: glycine, alanine, valine, leucine and isoleucine: 2) aliphatic hydroxyl side chain: serine and threonine: 3) amide-containing side chain: asparagine and glutamine: 4) aromatic side chain: phenylalanine, tyrosine and tryptophan: 5) basic side chain: lysine, arginine and histidine: 6) acidic side chain: aspartic acid and glutamic acid; and 7) sulfur-containing side chain: cysteine and methionine. For example, the conservative amino acid substitution group is valine-leucine-isoleucine-glycine-alanine, phenylalanine-tyrosine, threonine-serine, lysine-arginine, glutamic acid-aspartic acid and asparagine-glutamine.

Optionally, the conservative substitution is any changes with a positive value in a PAM250 log-likelihood matrix disclosed in Gonnet et al., Science 256:1443-45 (1992) (incorporated herein by reference). A "moderately conservative" substitution is any changes with a non-negative value in the PAM250 log-likelihood matrix.

### Brief Description of the Drawings

Fig. 1: effects of different concentrations of PD-1-112-C2 on IL-2/IFN-γ secretion.
Fig. 2: effects of different concentrations of PD-1-97-C2 on IL-2/IFN-γ secretion.
Fig. 3: effects of different concentrations of PD-1-76-C2 on IL-2/IFN-γ secretion.
Fig. 4: effects of different concentrations of PD-1-97-C2 on T cell proliferation and a cytokine IL-2 secreted by T cells.
Fig. 5: effects of different concentrations of PD-1-112-C2 on T cell proliferation and the cytokine IL-2 secreted by T cells.
Fig. 6: effects of different concentrations of PD-1-76-C2 on T cell proliferation and the cytokine IL-2 secreted by T cells.
Fig. 7: effects of different concentrations of PD-1-97-C2 on T cell proliferation and a cytokine IFN-γ secreted by T cells.
Fig. 8: effects of different concentrations of PD-1-112-C2 on T cell proliferation and the cytokine IFN-γ secreted by T cells.
Fig. 9: effects of different concentrations of PD-1-76-C2 on T cell proliferation and the cytokine IFN-γ secreted by T cells.

### Detailed Description of the Embodiments

Implementation schemes of the present invention are described in detail below in combination with embodiments, but those skilled in the art may understand that the following embodiments are only used to describe the present invention, and should not be regarded as limiting a scope of the present invention. If a specific condition is not indicated in the embodiment, it is performed according to a conventional condition or a condition suggested by a manufacturer. Reagents or instruments used without the indications of manufactures are all conventional products that may be obtained by market purchase.

### Embodiment 1: Preparation of anti-PD-1 antibody

### 1. Immunogen

A human PD-1 sequence (NCBI NP 005009), an upstream primer CCGCAAGCTTGCCGCCACCATG (SEQ ID NO: 1) and a downstream primer CCGGAATTCTCATTAATGGTGATGGTGATGATGCTGGAACTGGCCGGCAGGTC (SEQ ID NO: 2) were artificially synthesized, an extracellular domain was amplified through polymerase chain reaction (PCR) and cloned into a pCDNA3.4A eukaryotic expression system after double digestion with Hind III and EcoRI, then 293 cells were transfected with this plasmid, and the supernatant was harvested and purified to obtain recombinant human PD-1 recombinant protein (hPD-1).

### 2. Immunize animal

The recombinant hPD-1 protein at a concentration of 1.23 mg/ml was used as an antigen. Totally 125µg recombinant PD1 was mixed with the same amount of an immune adjuvant of Freund's adjuvant (Sigma-Aldrich F5881). 5 6-week-old female BAL b/C mice were subcutaneously immunized, and the amount of the immunized antigen for each mouse was 25 µg. After primary immunization, booster immunization of the same dose was performed once a week. After a total of 5 immunizations, the immune response was monitored by collecting mouse tail blood and testing serum titer. After fluorescence activating cell sorter (FACS) screening (as described below), the mice with sufficient anti-hPD-1 immunoglobulin titers were used for fusion. Three days after the intraperitoneal booster immunization with the antigen, the mice were killed and spleens were removed for hybridoma fusion.

### 3. Selection of BAL b/C mice producing anti-hPD-1 antibody

In order to select the BAL b/C mice producing the anti-hPD-1 antibody, immunized mouse serum was tested by FACS. Serum dilutions from the mice immunized with the hPD-1 recombinant protein were incubated with hPD1-transfected CHO cells at 4°C for 30 minutes, and after 3 times washing with phosphate buffered saline (PBS), 0.4 µg/ml of PE goat anti-mouse IgG (Biolegend 405307) was added and incubated at 4°C for another 30 minutes. After 3 times washing with PBS, a samples were load into a Beckman Coulter flow cytometer (CytoFLEX A00-1-1102) to verify whether the antibodies in samples may bind to the hPD1-transfected CHO cells, the BAL b/C mice producing the anti-hPD-1 antibody were selected, and the cell fusion was performed.

### 4. Generation of hybridoma producing mouse monoclonal antibody against hPD-1

Spleen cells of the immunized BAL b/C mice were fused with mouse myeloma cells, and the obtained hybridomas were screened for the antigen-specific antibody. Single cell suspension of the spleen cells from the immunized mice was fused at a ratio 5:1 with the mouse myeloma cells (SP2/0, ATCC CRL1581) in the presence of polyethylene glycol (PEG) 1500 (Roche 10783641001). The mouse myeloma cells did not secrete immunoglobulins before cell fusion. The fused cells were plated into a 96-well cell culture plate at about 1×10⁵ cells/well, All cells were grown in a 37°C incubator (Panasonic MCO-18AIC) supplied with 5% CO₂. Subsequently, the cells were cultured for about one week in an HAT selective culture medium which contains 1X penicillin-streptomycin dual antibody (Gibco 15140122), 1X HAT (Sigma CRLP-7185) and 20% fetal bovine serum (Royacel RY-F11-01) in a 1640 culture medium. After 1 week, the HAT culture medium was replaced with an HT culture medium (the 1640 culture medium containing 1X penicillin-streptomycin dual antibody (gibco 15140122), 1X HT (gibco 11067030) and 20% fetal bovine serum (Royacel RY-F11-01)) for culture, and then cell culture supernatants of the fusion plate was detected by FACS.The hybridomas secreting the antibody that may bind to the hPD-1 protein were selected. replated, for another round screening. The positive hybridomas that secreting hPD1 binding antibodies were subcloned at least twice by limiting dilution. Stable subclones were then cultured in vitro and a small amount of the antibodies were generated for further analysis. In this case, hybridoma clones PD1-112-C2, PD1-97-C2, and PD-76-C2 were selected for next evaluation.

### Embodiment 2: Affinity characterization of anti-PD-1 mouse monoclonal antibody

According to a conventional method, a Chinese hamster ovary cell (CHO) cell line (CHO-hPD1) expressing recombinant human PD-1 on the cell surface, a CHO cell line (CHO-cynoPD1) expressing monkey PD1 (Uniprot: BOLAJ2), and a CHO cell line (CHO-mousePD1) expressing mouse PD1 (Uniprot: Q02242) were prepared by a recombinant technology, and these three cell lines were used in flow cytometry(FCM) for measuring binding characterization of anti-PD-1 mouse monoclonal antibodies PD-1-76-C2, PD-1-97-C2, and PD-1-112-C2 measured by a flow cytometry (FCM).

In order to evaluate binding of the anti-PD-1 mouse monoclonal antibody to CHO-hPD1, 2×10⁵ CHO-hPD1 cells and the anti-PD-1 mouse monoclonal antibody that diluted in a concentration gradient (the initial concentration was 10 µg/ml, 3-fold serial dilution) were added to a 96-well plate. then it was incubated at 4°C for 30 minutes. After the cells were washed once with a buffer (PBS containing 3% bovine serum albumin (BSA)), PE-labeled anti-mouse IgG(Fc) Ab (Biolegend) fluorescent secondary antibody was added, it was incubated at 4°C for another 30 minutes. the cells were washed once with the buffer and resuspended in PBS, and then cell suspension was analyzed through flow cytometry by CytoFlex (Beckman flow cytometer), the amount of the antibody bound to the cells was measured according to the mean fluorescence intensity (MFI); the same method was used to evaluate binding of anti-PD-1 mouse monoclonal antibody to a CHO-cyno cell, and CHO-mousePD1 (sometimes abbreviated as "CHO-mPD1" in the present invention) cell. Results were shown in Table 1, data showed that the anti-PD-1 mouse monoclonal bodies PD-1-76-C2, PD-1-97-C2, and PD-1-112-C2 may all bind to the CHO-hPD1 cell and CHO-cyno cell with high affinity; while all of three mouse monoclonal bodies may not bind to the CHO-mousePD1 cell (data omitted).

### Embodiment 3: Binding of PD-1 antibody to activated PBMC

Fresh human peripheral blood mononuclear cells (PBMC) could activate and proliferate lymphocytes under the stimulation of PHA (Sigma), and express PD1 with the highest abundance on the third day, so it may be used for a binding experiment of PD-1 antibody and PD1 naturally expressed by the activated lymphocytes.

After PBMC was obtained from fresh human peripheral blood by a lymphatic separation fluid gradient centrifugation method, the density was adjusted to 1×10⁶ cells/ml and then inoculated into T75. At the same time, PHA-L (Sigma) at a final concentration of 1 µg/ml was added to stimulate lymphocyte proliferation. After standing in an incubator supplied with 37°C and 5% CO₂ for 3 days, cell suspension was taken out and centrifuged to remove the supernatant. Cells were resuspended in a buffer (PBS containing 3% BSA), and added into a 96-well U-shaped plate at a density of 2*10⁵ cells/well. And then the anti-PD1 antibody that prepared for a total of 10 concentration gradients (started from 30 µg/ml and diluted in a 3-fold gradient) was added. After incubation at 4°C for 30 minutes, the plate was centrifuged at 300 g for 5 minutes, cells were washed once with the buffer. A PE-labeled goat anti-human IgG fluorescent antibody (Biolegend) was added, and incubated at 4°C for 30 minutes; after incubation, the cells were washed once by centrifugation resuspended in PBS, and analyzed by a CytoFlex flow cytometer to detect the amount of the antibody bound to PBMC. Results were shown in Table 1. The anti-PD1 antibody may bind to the activated lymphocytes with high affinity.

### Embodiment 4: Binding specificity of anti-PD-1 mouse monoclonal antibody

To verify the specificity of the antibody binding to PD-1, the binding assay of anti-PD-1 mouse monoclonal antibody with four different CD28 family member proteins was conducted. According to standard enzyme-linked immunosorbent assay (ELISA) method, PD-1, CD28, CTLA-4, and ICOS (ACRO) were coated at a concentration of 1 µg/ml on an ELISA plate, and the anti-human PD-1 mouse monoclonal antibody at a concentration of 10 µg/ml was added. Anti-mouse IgG coupled with peroxidase (HRP) was used as a secondary antibody (Sigma). The color was developed by TMB, and after reaction termination, the absorbance was read by a microplate reader. Results were shown in Table 2. The anti-PD-1 mouse monoclonal antibodies PD-1-76-C2, PD-1-97-C2 and PD-1-112-C2 may all specifically bind to PD-1, but not other family members of CD28.

### Embodiment 5: Determination of anti-human PD-1 mouse monoclonal antibody affinity by biolayer interference (BLI) method

ForteBio (Octet Qke) affinity determination: by loading a PD-1-his (ACRO) recombinant protein at a concentration of 5 µg/ml on an HISIK biosensor for 120 seconds, and then equilibrating the loaded sensor in a standard buffer (PBST, PBS+0.02% Tuween20) for 120 seconds, after that, the sensor was transferred to an anti-PD-1 mouse monoclonal antibody dilution for 180 seconds to measure the binding rate, and then transferred to the standard buffer for 20 minutes to measure the dissociation rate. Finally, a kinetic model was used for analysis, and processed data was shown in Table 3.

### Embodiment 6: Anti-PD-1 mouse monoclonal antibody blocks the binding of ligand PD-L1/PD-L2 to CHO-hPD1

The ability of the anti-PD-1 mouse monoclonal antibody to block the binding of the ligand to HO-hPD1 cells was analyzed by a flow cytometer. The ligand proteins used in the experiment were fusion proteins that consists of recombinant PD-L1/PD-L2 extracellular domain and human IgG1 Fc fragment fusion proteins: PD-L1-hFc (ACRO), and PD-L2-hFc (ACRO).

CHO-hPD1 cells were resuspended in a buffer (PBS containing 3% BSA), the density was adjusted to 2×10⁶ cells/ml,then cell suspension was added to a 96-well U-shaped plate in 100 µl/well, The plate was centrifuged at 300 g for 5 minutes, and the supernatant was removed.

The subsequent process may be divided into two blocking modes: mode 1, PD-L1-hFc/PD-L2-hFc at a concentration of 3 µg/ml was added to cell wells, and incubated at 4°C for 30 minutes, and then the anti-PD1 antibody that prepared for a total of 10 concentration gradients (started from 30 µg/ml, diluted in a 3-fold gradient) was added, the anti-PD1 antibody that prepared for a total of 10 concentration gradients (started from 30 µg/ml, diluted in a 3-fold gradient) was added to cell wells, after incubation at 4°C for 30 minutes, the PD-L1-hFc/PD-L2-hFc protein at a concentration of 3 µg/ml was added, and incubated at 4°C for 30 another minutes.

The plate was centrifuged at 300 g for 5 minutes, cells were washed once with a buffer, a PE-labeled goat anti-human IgG fluorescent antibody (Biolegend) was added, and incubated at 4°C for 30 minutes. After the cells were washed once by centrifugation, the cells were resuspended in PBS, and then analyzed by a CytoFlex flow cytometer, to detect the amount of the ligand protein bound to the cells, and the IC₅₀ value of the PD-1 antibody binding blocking was calculated. Results were shown in Table 4, and three anti-PD-1 mouse monoclonal antibodies: PD-1-76-C2, PD-1-97-C2, and PD-1-112-C2 may all effectively block the binding of PD-L1/PD-L2 to cell CHO-PD1.

### Embodiment 7: Effect of anti-PD-1 antibody on cytokine release from SEB-stimulated PBMC cell

In this embodiment, PBMCs cultured overnight were stimulated by addition of superantigen staphylococcus aureus enterotoxin B (SEB), in the presence or absence of the anti-PD-1 antibody, the effect of secretion of cytokines was detected.

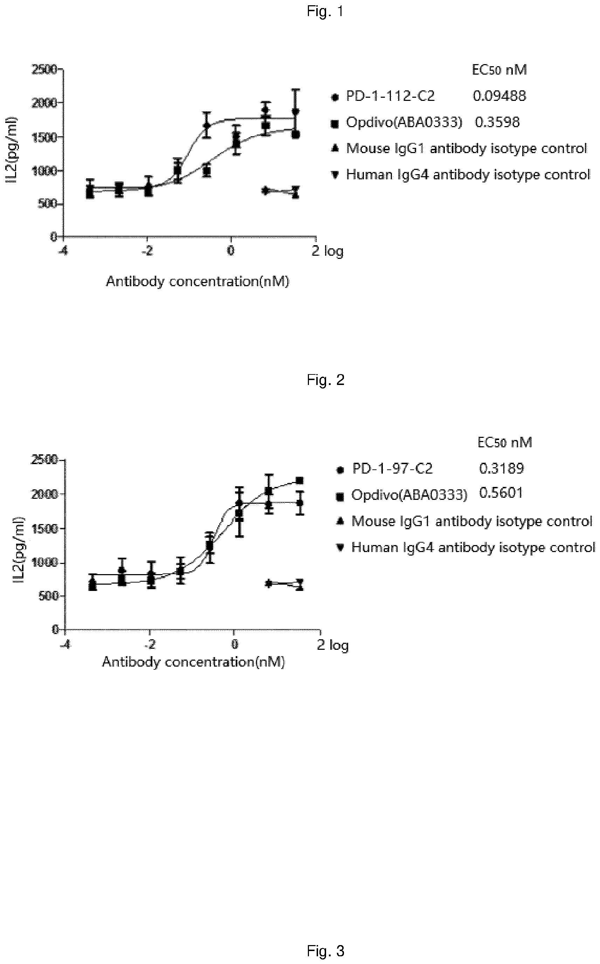

After fresh PBMCs were resuspended in an X-VIVO 15 culture medium (LONZA) containing 10% FBS. The cells were added to a T25 culture flask, and cultured stationarily overnight at 37°C and 5% CO₂. Suspended cells were taken on the next day, and after being centrifuged, they were resuspended in a fresh X-VIVO (containing 10% FBS) culture medium, and added to a 96-well flat plate with a density of 1×10⁵ cells per well. And then a SEB superantigen (Toxin technology) at a final concentration of 200 ng/ml was added. At the same time, the anti-PD-1 antibodies of different concentrations were added, isotype control (mlgG1 isotype control antibody (Biolegend); and hlgG4 isotype control antibody (Biolegend)) and negative control with no antibody were set additionally. After 3 days, constant supernatants were taken from a sample wells, and the level of IL-2/IFN-γ was measured with an IL2/IFN-γ Human Uncoated ELISA Kit (eBioscience), and results were shown in Figs. 1-3, the secretion of IL-2/IFN-γ was improved by the anti-PD-1 antibody in a concentration-dependent manner. These results indicated that in SEB superantigen-stimulated PBMCs, the anti-PD-1 antibodies: PD-1-76-C2, PD-1-97-C2, and PD-1-112-C2 could further promote T cells to secrete the cytokines.

### Embodiment 8: Effect of anti-PD-1 antibody in mixed lymphocyte reaction

In the mixed lymphocyte reaction (MLR), the presence or absence of the anti-PD-1 antibody can demonstrate the proliferation of T cells and the level of cytokines secreted by T cells when the PD1 signal is blocked.

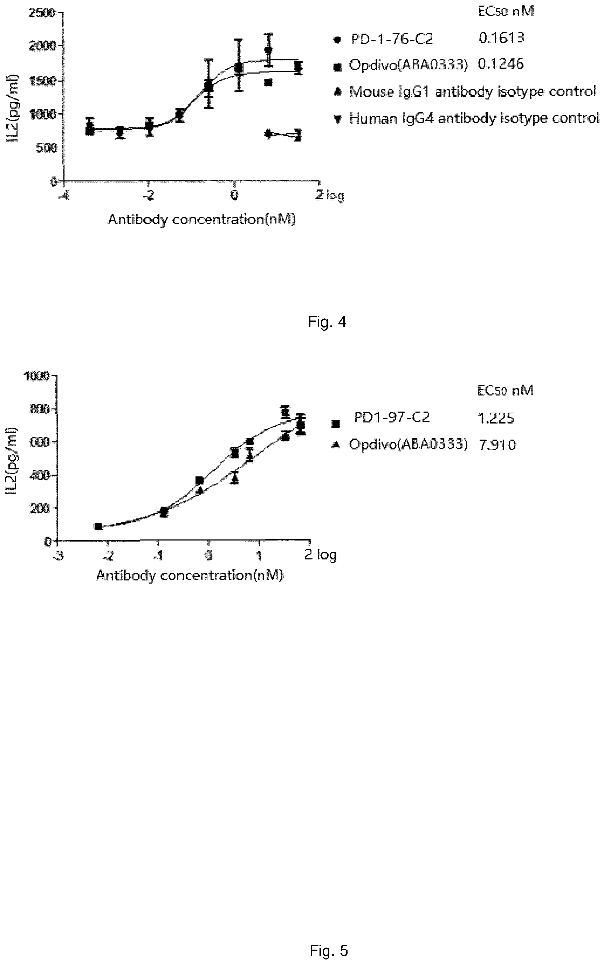

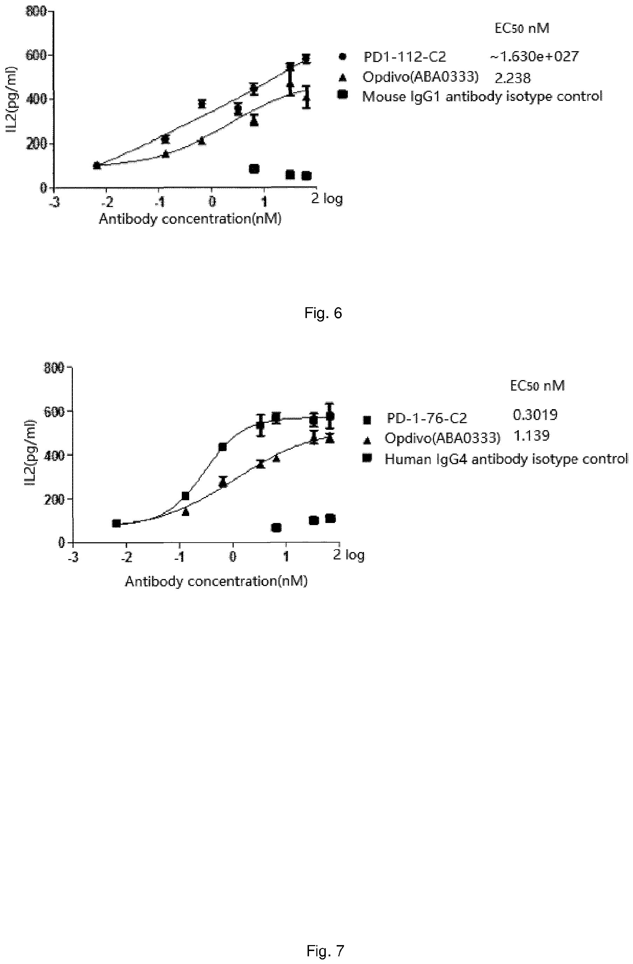

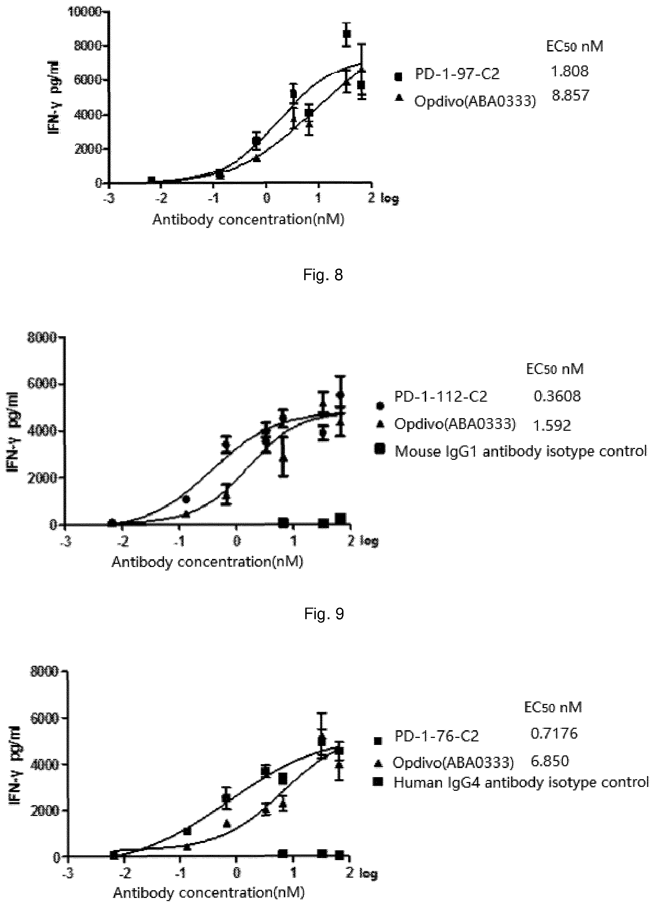

CD14⁺ monocytes were isolated from fresh PBMCs with CD14 MicroBeads, human (Miltenyi). In the presence of GM-CSF/IL-4, after 6 days of induction, TNF-α was added, and 3 days later, immature dendritic cells (DC) were induced to mature DC; On the day of the experiment, T cells in PBMCs were isolated with EasySep^{™} Human T Cell Enrichment Kit (StemCell), 1×10⁴ DC cells were mixed and cultured with 1×10⁵ T cells, and different concentration gradients of anti-PD-1 antibodies were added to the mixed cells. Isotype control antibodies (mlgG1 isotype control antibody and hIgG4 isotype control antibody (Biolegend)) and no antibody control well were set additionally. After 3 days of mixed culture, supernatants were taken for detection of IL-2 production, and at day 5, the supernatants were taken for detection of IFN-γ production. Results were shown in Figs. 4-9, the anti-PD-1 antibodies: PD-1-76-C2, PD-1-97-C2, and PD-1-112-C2 in the MLR experiment, may block the binding of PD1 to the ligand in an antibody concentration-dependent mode, and inhibit the PD1 signaling pathway, thereby the proliferation of T cells was promoted, and the secretion of IL-2 and IFN-γ was promoted.

### Embodiment 9: Candidate antibody variable region sequence

Candidate antibody hybridoma cells were cultured to 1*10⁷ cells, it was centrifuged at 300 g for 10 minutes, and RNA was extracted using an MagExtractor ^{®}-RNA-kit (Toyobo/NPK-201F). The RNA was denatured and then reverse-transcribed to obtain cDNA. PCR was performed separately for heavy and light chains with the cDNA as a template. A Light Chain gene was amplified with Universal Primer A Mix (UPM), Nested Universal Primer A (NUP) and mkR primers, and a Heavy Chain gene was amplified with Universal Primer A Mix (UPM), Nested Universal Primer A (NUP) and mHR primers. Herein, the primer pair of Light Chain amplifies a target band of about 0.8 KB, and the primer pair of Heavy Chain amplifies a target band of about 1.4 KB.
Universal Primer A Mix (UPM):
   5'>CTAATACGACTCACTATAGGGCAAGCAGTGGTATCAACGCAGAGT<3' (SEQ ID NO: 3);
Nested Universal Primer A (NUP):
   5' > AAGCAGTGGTATCAACGCAGAGT < 3' (SEQ ID NO: 4)
mkR: 5'>TTTTCCTTTTGAATTCCTAACACTCATTCCTGTTGAAGC<3' (SEQ ID NO: 5);
mHR: 5'>TTTTCCTTTTGAATTCTCATTTACCAGGAGAGTGGGA<3' (SEQ ID NO: 6).

The total reaction volume was 50 µL, containing 25 µL of PCR 2x buffer, 1 µL of DNA template, 1 µL of KOD DNA Polymerase, 10 µL of dNTP (25 mM), and 1 µL of each specific upper or lower primer (the final concentration is 200 nmol/L), and sterilized double distilled water was added to a total volume of 50 µL.

The reaction conditions were pre-denaturation at 94°C for 5 minutes, followed by denaturation at 94°C for 30 seconds, annealing at 56°C for 30 seconds, and extension at 72°C for 40 seconds, and there was a total of 30 cycles.

After sequencing amplified products, variable regions of the heavy and light chains of hybridoma clones 76, 97 and 112 were obtained as follows:
PD-1-76-C2
   Heavy chain variable region: SEQ ID NO: 7
   HCDR1: SEQ ID NO: 8
   HCDR2: SEQ ID NO: 9
   HCDR3: SEQ ID NO: 10
PD-1-97-C2
   Heavy chain variable region: SEQ ID NO: 11
   HCDR1: SEQ ID NO: 12
   HCDR2: SEQ ID NO: 13
   HCDR3: SEQ ID NO: 14
PD-1-112-C2
   Heavy chain variable region: SEQ ID NO: 15
   HCDR1: SEQ ID NO: 16
   HCDR2: SEQ ID NO: 17
   HCDR3: SEQ ID NO: 18
PD-1-76-C2
   Light chain variable region: SEQ ID NO: 19
   LCDR1: SEQ ID NO: 20
   LCDR2: SEQ ID NO: 21
   LCDR3: SEQ ID NO: 22
PD-1-97-C2
   Light chain variable region: SEQ ID NO: 23
   LCDR1: SEQ ID NO: 24
   LCDR2: SEQ ID NO: 25
   LCDR3: SEQ ID NO: 26
PD-1-112-C2
   Light chain variable region: SEQ ID NO: 27
   LCDR1: SEQ ID NO: 28
   LCDR2: SEQ ID NO: 29
   LCDR3: SEQ ID NO: 30

**Table 1**

| Antibody to be tested | FCM, EC₅₀, nM | | |
|---|---|---|---|
| | CHO-hPD1 | CHO-cynoPD1 | PBMC (activated) |
| PD-1-76-C2 | 0.583 | 0.576 | 0.02 |
| PD-1-97-C2 | 0.745 | 0.578 | 1.033 |
| PD-1-112-C2 | 0.739 | 0.463 | 0.109 |

**Table 2**

| Antibody to be tested | Elisa, OD value | | | |
|---|---|---|---|---|
| | Human PD-1-hFc | Human CD28-hFc | Human ICOS-hFc | Human CTLA4-hFc |
| PD-1-76-C2 | 2.457 | 0.0616 | 0.076 | 0.1457 |
| PD-1-97-C2 | 2.371 | 0.0606 | 0.0636 | 0.1293 |
| PD-1-112-C2 | 2.1213 | 0.0625 | 0.062 | 0.131 |

**Table 3**

| Antibody to be tested | Kon (1/Ms) | Kdis (1/s) | KD (M) |
|---|---|---|---|
| Opdivo (ABA0333) | 1.38E+06 | 3.63E-06 | 2.62E-12 |
| PD-1-97-C2 | 4.68E+05 | <1.0E-07 | <1.0E-12 |
| PD-1-112-C2 | 7.32E+05 | 3.18E-05 | 4.35E-11 |
| PD-1-76-C2 | 7.71E+05 | <1.0E-07 | <1.0E-12 |

**Table 4**

| Antibody to be tested | FCM, IC₅₀, nM | | | |
|---|---|---|---|---|
| | Blocking CHO-hPD1 and PD-L1 | | Blocking CHO-hPD1 and PD-L2 | |
| | Mode 1 | Mode 2 | Mode 1 | Mode 2 |
| PD-1-76-C2 | 2.28 | 1.4 | 4.669 | 3.112 |
| PD-1-97-C2 | 2.218 | 1.621 | 7.539 | 3.493 |
| PD-1-112-C2 | 2.567 | 1.546 | 4.614 | 3.727 |

## Claims

1. An anti-PD1 antibody or an antigen-binding fragment thereof, comprising a heavy chain CDR1-3 and a light chain CDR1-3 selected from a group consisting of the following items:
(1) HCDR1: represented by a sequence of SEQ ID NO: 8; HCDR2: represented by a sequence of SEQ ID NO: 9; HCDR3: represented by a sequence of SEQ ID NO: 10;
LCDR1: represented by a sequence of SEQ ID NO: 20; LCDR2: represented by a sequence of SEQ ID NO: 21; LCDR3: represented by a sequence of SEQ ID NO: 22;
(2) HCDR1: represented by a sequence of SEQ ID NO: 12; HCDR2: represented by a sequence of SEQ ID NO: 13; HCDR3: represented by a sequence of SEQ ID NO: 14;
LCDR1: represented by a sequence of SEQ ID NO: 24; LCDR2: represented by a sequence of SEQ ID NO: 25; LCDR3: represented by a sequence of SEQ ID NO: 26; and
(3) HCDR1: represented by a sequence of SEQ ID NO: 16; HCDR2: represented by a sequence of SEQ ID NO: 17; HCDR3: represented by a sequence of SEQ ID NO: 18;
LCDR1: represented by a sequence of SEQ ID NO:28; LCDR2: represented by a sequence of SEQ ID NO:29; LCDR3: represented by a sequence of SEQ ID NO:30.

2. The antibody or antigen-binding fragment thereof according to claim 1, wherein the antibody comprises:
(1) a heavy chain variable region, which comprises or consists of the following sequences:
an amino acid sequence shown in SEQ ID NO: 7, or
a sequence having at least 60%, 70%, 80%, and 85%, preferably at least 86%, 87%, 88%, 89%, 90%, 91%, 92%, 93%, 94%, 95%, 96%, 97%, 98% or 99% of the sequence identity with the sequence shown in SEQ ID NO: 7, and
a light chain variable region, which comprises or consists of the following sequences:
an amino acid sequence shown in SEQ ID NO: 19, or
a sequence having at least 60%, 70%, 80%, and 85%, preferably at least 86%, 87%, 88%, 89%, 90%, 91%, 92%, 93%, 94%, 95%, 96%, 97%, 98% or 99% of the sequence identity with the sequence shown in SEQ ID NO: 19;
(2) a heavy chain variable region, which comprises or consists of the following sequences:
an amino acid sequence shown in SEQ ID NO: 11, or
a sequence having at least 60%, 70%, 80%, and 85%, preferably at least 86%, 87%, 88%, 89%, 90%, 91%, 92%, 93%, 94%, 95%, 96%, 97%, 98% or 99% of the sequence identity with the sequence shown in SEQ ID NO: 11, and
a light chain variable region, which comprises or consists of the following sequences:
an amino acid sequence shown in SEQ ID NO: 23, or
a sequence having at least 60%, 70%, 80%, and 85%, preferably at least 86%, 87%, 88%, 89%, 90%, 91%, 92%, 93%, 94%, 95%, 96%, 97%, 98% or 99% of the sequence identity with the sequence shown in SEQ ID NO: 23; and
(3) a heavy chain variable region, which comprises or consists of the following sequences:
an amino acid sequence shown in SEQ ID NO: 15, or
a sequence having at least 60%, 70%, 80%, and 85%, preferably at least 86%, 87%, 88%, 89%, 90%, 91%, 92%, 93%, 94%, 95%, 96%, 97%, 98% or 99% of the sequence identity with the sequence shown in SEQ ID NO: 15, and
a light chain variable region, which comprises or consists of the following sequences:
an amino acid sequence shown in SEQ ID NO: 27, or
a sequence having at least 60%, 70%, 80%, and 85%, preferably at least 86%, 87%, 88%, 89%, 90%, 91%, 92%, 93%, 94%, 95%, 96%, 97%, 98% or 99% of the sequence identity with the sequence shown in SEQ ID NO: 27.

3. The antibody or antigen-binding fragment thereof according to any one of claims 1-2, wherein the antibody further comprises a heavy chain constant region and a light chain constant region, preferably derived from human IgG or IgM, more preferably IgG4.

4. The antibody or antigen-binding fragment thereof according to any one of claims 1-2, wherein the antigen-binding fragment is selected from Fab, scFv, Fab', dAb, F(ab')₂, Fv or Fab/c.

5. The antibody or antigen-binding fragment thereof according to any one of claims 1-2, wherein the antibody is a chimeric antibody or a multispecific antibody.

6. A polynucleotide encoding the antibody or antigen-binding fragment thereof according to any one of claims 1-5.

7. A pharmaceutical composition, comprising the antibody or antigen-binding fragment thereof according to any one of claims 1-5; and optionally, which further comprises a pharmaceutically acceptable carrier and/or excipient.

8. Use of the PD-1 antibody or antigen-binding fragment thereof according to any one of claims 1-5, or the pharmaceutical composition according to claim 7 in preparation of a drug for treating a PD-1-mediated disease or disorder.

9. Use of the antibody or antigen-binding fragment thereof according to any one of claims 1-5, or the pharmaceutical composition according to claim 7 in prevention and/or treatment and/or adjuvant treatment and/or diagnosis treatment of a tumor disease or in preparation of a drug for the prevention and/or treatment and/or adjuvant treatment and/or diagnosis treatment of the tumor disease.

10. The pharmaceutical composition according to claim 7 or the use according to claim 9, wherein the pharmaceutical composition or drug is in a form suitable for injection, preferably the form suitable for administration by subcutaneous injection, intradermal injection, intravenous injection, intramuscular injection or intralesional injection.


SEQUENCE LISTING

<110>  SHENZHEN FEIPENG BIOLOGICAL PHARMACEUTICAL CO. LTD

<120>  Anti-human programmed death-1 monoclonal antibody

<130>  PN183096FPSW

<150>  CN201911342193.8
<151>  2019-12-20

<160>  30

<170>  PatentIn version 3.5

<210>  1
<211>  22
<212>  DNA
<213>  Artificial Sequence

<220>
<223>  Synthesized

<400>  1
ccgcaagctt gccgccacca tg                                                22


<210>  2
<211>  53
<212>  DNA
<213>  Artificial Sequence

<220>
<223>  Synthesized

<400>  2
ccggaattct cattaatggt gatggtgatg atgctggaac tggccggcag gtc              53


<210>  3
<211>  45
<212>  DNA
<213>  Artificial Sequence

<220>
<223>  Synthesized

<400>  3
ctaatacgac tcactatagg gcaagcagtg gtatcaacgc agagt                       45


<210>  4
<211>  23
<212>  DNA
<213>  Artificial Sequence

<220>
<223>  Synthesized

<400>  4
aagcagtggt atcaacgcag agt                                               23

<210>  5
<211>  39
<212>  DNA
<213>  Artificial Sequence

<220>
<223>  Synthesized

<400>  5
ttttcctttt gaattcctaa cactcattcc tgttgaagc                              39


<210>  6
<211>  39
<212>  DNA
<213>  Artificial Sequence

<220>
<223>  Synthesized

<400>  6
ttttcctttt gaattctcat ttaccaggag agtgggaga                              39


<210>  7
<211>  118
<212>  PRT
<213>  Artifical Sequence

<400>  7

Gln Ile Gln Leu Val Gln Ser Gly Pro Glu Leu Lys Lys Pro Gly Glu
1               5                   10                  15


Thr Val Lys Ile Ser Cys Lys Ala Ser Gly Tyr Thr Phe Thr Asn Tyr
            20                  25                  30


Gly Met Asn Trp Val Lys Gln Ala Pro Gly Lys Gly Leu Lys Trp Met
        35                  40                  45


Gly Trp Ile Asn Thr His Thr Gly Glu Pro Thr Tyr Ala Asp Asp Phe
    50                  55                  60


Lys Gly Arg Phe Ala Phe Ser Leu Glu Thr Ser Ala Ser Thr Ala Tyr
65                  70                  75                  80


Leu Gln Ile Lys Asn Leu Lys Asn Glu Asp Val Ala Thr Tyr Phe Cys
                85                  90                  95


Thr Lys Glu Gly Glu Gly Ile Gly Phe Ala Tyr Trp Gly Gln Gly Thr
            100                 105                 110


Leu Val Thr Val Ser Ala
        115

<210>  8
<211>  5
<212>  PRT
<213>  Artificial Sequence

<220>
<223>  Synthesized

<400>  8

Asn Tyr Gly Met Asn
1               5


<210>  9
<211>  17
<212>  PRT
<213>  Artificial Sequence

<220>
<223>  Synthesized

<400>  9

Trp Ile Asn Thr His Thr Gly Glu Pro Thr Tyr Ala Asp Asp Phe Lys
1               5                   10                  15


Gly


<210>  10
<211>  9
<212>  PRT
<213>  Artificial Sequence

<220>
<223>  Synthesized

<400>  10

Glu Gly Glu Gly Ile Gly Phe Ala Tyr
1               5


<210>  11
<211>  118
<212>  PRT
<213>  Artificial Sequence

<220>
<223>  Synthesized

<400>  11

Glu Val Lys Leu Val Glu Ser Gly Gly Gly Leu Val Lys Pro Gly Gly
1               5                   10                  15


Ser Leu Lys Leu Ser Cys Ala Ala Ser Gly Phe Thr Phe Ser Ser Tyr
            20                  25                  30

Gly Met Ser Trp Ile Arg Gln Thr Pro Glu Lys Arg Leu Glu Trp Val
        35                  40                  45

Ala Thr Ile Ser Gly Gly Gly Arg Asp Ser Tyr Tyr Pro Asp Ser Met
    50                  55                  60

Lys Gly Arg Phe Ala Ile Ser Arg Asp Asn Ala Lys Asn Asn Leu Tyr
65                  70                  75                  80

Leu Gln Met Ser Ser Leu Arg Ser Glu Asp Thr Ala Leu Tyr Tyr Cys
                85                  90                  95

Thr Arg Gln Leu Gly Pro Ala Trp Phe Ala Tyr Trp Gly Gln Gly Thr
            100                 105                 110

Leu Val Thr Val Ser Ala
        115

<210>  12
<211>  5
<212>  PRT
<213>  Artificial Sequence

<220>
<223>  Synthesized

<400>  12

Ser Tyr Gly Met Ser
1               5

<210>  13
<211>  17
<212>  PRT
<213>  Artificial Sequence

<220>
<223>  Synthesized

<400>  13

Thr Ile Ser Gly Gly Gly Arg Asp Ser Tyr Tyr Pro Asp Ser Met Lys
1               5                   10                  15

Gly

<210>  14
<211>  9
<212>  PRT
<213>  Artificial Sequence

<220>

<223>  Synthesized

<400>  14

Gln Leu Gly Pro Ala Trp Phe Ala Tyr
1               5

<210>  15
<211>  120
<212>  PRT
<213>  Artificial Sequence

<220>
<223>  Synthesized

<400>  15

Gln Val Gln Leu Gln Gln Pro Gly Ala Glu Leu Val Lys Pro Gly Ala
1               5                   10                  15

Ser Val Lys Leu Ser Cys Lys Ala Ser Gly Tyr Thr Phe Thr Thr Tyr
            20                  25                  30

Tyr Met Tyr Trp Val Lys Gln Arg Pro Gly Gln Gly Leu Glu Trp Ile
        35                  40                  45

Gly Gly Ile Asn Pro Ser Asn Gly Gly Thr Asn Phe Asn Glu Lys Phe
    50                  55                  60

Lys Ser Lys Ala Thr Leu Thr Val Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Thr Arg Arg Asp Ser Asn Tyr Asp Gly Gly Phe Asp Tyr Trp Gly Gln
            100                 105                 110

Gly Thr Thr Leu Thr Val Ser Ser
        115                 120

<210>  16
<211>  5
<212>  PRT
<213>  Artificial Sequence

<220>
<223>  Synthesized

<400>  16

Thr Tyr Tyr Met Tyr
1               5

<210>  17
<211>  17
<212>  PRT
<213>  Artificial Sequence

<220>
<223>  Synthesized

<400>  17

Gly Ile Asn Pro Ser Asn Gly Gly Thr Asn Phe Asn Glu Lys Phe Lys
1               5                   10                  15

Ser

<210>  18
<211>  11
<212>  PRT
<213>  Artificial Sequence

<220>
<223>  Synthesized

<400>  18

Arg Asp Ser Asn Tyr Asp Gly Gly Phe Asp Tyr
1               5                   10

<210>  19
<211>  113
<212>  PRT
<213>  Artificial Sequence

<220>
<223>  Synthesized

<400>  19

Asp Val Leu Met Thr Gln Thr Pro Leu Ser Leu Pro Val Ser Leu Gly
1               5                   10                  15

Asp Gln Ala Ser Ile Ser Cys Arg Ser Ser Gln Ser Ile Val Tyr Ser
            20                  25                  30

Asn Gly Lys Thr Tyr Leu Glu Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Phe Gln Gly
                85                  90                  95

Ser His Val Pro Asn Thr Phe Gly Gly Gly Thr Lys Leu Glu Ile Lys
            100                 105                 110

Arg

<210>  20
<211>  16
<212>  PRT
<213>  Artificial Sequence

<220>
<223>  Synthesized

<400>  20

Arg Ser Ser Gln Ser Ile Val Tyr Ser Asn Gly Lys Thr Tyr Leu Glu
1               5                   10                  15

<210>  21
<211>  7
<212>  PRT
<213>  Artificial Sequence

<220>
<223>  Synthesized

<400>  21

Lys Val Ser Asn Arg Phe Ser
1               5

<210>  22
<211>  9
<212>  PRT
<213>  Artificial Sequence

<220>
<223>  Synthesized

<400>  22

Phe Gln Gly Ser His Val Pro Asn Thr
1               5

<210>  23
<211>  112
<212>  PRT
<213>  Artificial Sequence

<220>
<223>  Synthesized

<400>  23

Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Arg Val Asp Asn Ser
            20                  25                  30

Gly Ile Ser Phe Met Asn Trp Phe Gln Gln Lys Pro Gly Gln Pro Pro
        35                  40                  45

Lys Leu Leu Ile Tyr Thr Ala Ser Asn Gln Gly Ser Gly Val Pro Asp
    50                  55                  60

Arg Phe Ser Gly Ser Gly Ser Gly Thr Asp Phe Ser Leu Asn Ile His
65                  70                  75                  80

Pro Val Glu Glu Asp Asp Thr Ala Met Tyr Phe Cys Gln Gln Ser Lys
                85                  90                  95

Glu Val Pro Trp Thr Phe Gly Gly Gly Thr Lys Leu Glu Ile Lys Arg
            100                 105                 110

<210>  24
<211>  15
<212>  PRT
<213>  Artificial Sequence

<220>
<223>  Synthesized

<400>  24

Arg Ala Ser Glu Arg Val Asp Asn Ser Gly Ile Ser Phe Met Asn
1               5                   10                  15

<210>  25
<211>  7
<212>  PRT
<213>  Artificial Sequence

<220>
<223>  Synthesized

<400>  25

Thr Ala Ser Asn Gln Gly Ser
1               5

<210>  26
<211>  9
<212>  PRT
<213>  Artificial Sequence

<220>

<223>  Synthesized

<400>  26

Gln Gln Ser Lys Glu Val Pro Trp Thr
1               5


<210>  27
<211>  112
<212>  PRT
<213>  Artificial Sequence

<220>
<223>  Synthesized

<400>  27

Asp Ile Val Leu Thr Gln Ser Pro Ala Ser Leu Ala Val Ser Leu Gly
1               5                   10                  15


Gln Arg Ala Thr Ile Ser Cys Arg Ala Ser Lys Ser Val Ser Thr Ser
            20                  25                  30


Gly Tyr Ser Tyr Met His Trp Tyr Gln Gln Lys Pro Gly Gln Pro Pro
        35                  40                  45


Lys Leu Leu Ile Tyr Leu Ala Tyr His Leu Glu Ser Gly Val Pro Ala
    50                  55                  60


Arg Phe Ser Gly Ser Gly Ser Gly Thr Asp Phe Thr Leu Asn Ile His
65                  70                  75                  80


Pro Val Glu Glu Glu Asp Ala Ala Thr Tyr Tyr Cys Gln His Ser Trp
                85                  90                  95


Glu Leu Pro Ile Thr Phe Gly Ser Gly Thr Lys Leu Glu Ile Lys Arg
            100                 105                 110


<210>  28
<211>  15
<212>  PRT
<213>  Artificial Sequence

<220>
<223>  Synthesized

<400>  28

Arg Ala Ser Lys Ser Val Ser Thr Ser Gly Tyr Ser Tyr Met His
1               5                   10                  15


<210>  29
<211>  7
<212>  PRT

<213>  Artificial Sequence

<220>
<223>  Synthesized

<400>  29

Leu Ala Tyr His Leu Glu Ser
1               5


<210>  30
<211>  9
<212>  PRT
<213>  Artificial Sequence

<220>
<223>  Synthesized

<400>  30

Gln His Ser Trp Glu Leu Pro Ile Thr
1               5